# EUROPEAN PATENT APPLICATION

(11) **EP 3 998 478 A1**
(43) Date of publication of application: **18.05.2022**
(21) Application number: 20841396.3
(22) Date of filing: 10.07.2020
(51) Int. Cl.: G01N 33/50, G01N 33/53

(54) **DEVELOPMENT OF BLOOD FIBROSIS MARKER FOR NON-ALCOHOLIC STEATOHEPATITIS**

(30) Priority: 12.07.2019 JP 2019129798
(71) Applicant: National University Corporation Hokkaido University, Sapporo-shi, Hokkaido 060-0808 (JP)
(72) Inventor: FURUKAWA, Jun-ichi, Sapporo-shi, Hokkaido 060-0808 (JP); SAKAMOTO, Naoya, Sapporo-shi, Hokkaido 060-0808 (JP); HANAMATSU, Hisatoshi, Sapporo-shi, Hokkaido 060-0808 (JP); OGAWA, Koji, Sapporo-shi, Hokkaido 060-0808 (JP); SUDA, Goki, Sapporo-shi, Hokkaido 060-0808 (JP)
(74) Representative: Schnappauf, Georg
(86) International application number: PCT/JP2020/027144
(87) International publication number: WO 2021/010349

(57) **Abstract**

A method for evaluating the progression of hepatic fibrosis in non-alcoholic steatohepatitis, said method comprising measuring the amount of a sugar chain having a structure represented by formula (I) and/or a precursor sugar chain of the biosynthesis of a sugar chain having a structure represented by formula (I) in a sample.

## Description

### Technical Field

The present invention relates to a marker for liver disease. Particularly, the present invention relates to a blood fibrosis marker for non-alcoholic steatohepatitis.

### Background Art

Since the progress of hepatic fibrosis in non-alcoholic steatohepatitis (NASH) leads to the appearance of hepatic cirrhosis or liver cancer, the early detection of fibrosis is a very important object. Although histological evaluation by liver biopsy is the most accurate for the evaluation of hepatic fibrosis, the histological evaluation imposes heavy burdens on patients, and has a risk such as infection. Although hyaluronic acid in blood (Non Patent Literature 1), IV type collagen, M2BPGi, and the like have been used as noninvasive evaluation of hepatic fibrosis (Non Patent Literature 2), these increase in the cases of other diseases. Therefore, a diagnostic marker having high specificity to a disease has therefore been desired. For example, M2BPGi has been discovered as an index for measuring the hepatic fibrosis of hepatitis C patients (Non Patent Literature 2), and it is not optimized for the determination of hepatic fibrosis in NASH. Although the inventors found that the expression of an N-linked sugar chain (A3F) on α1-antitrypsin in serum increased significantly in the progress of fibrosis in NASH disease in the past (Patent Literature 1), A3F exhibited correlation with inflammation in hepatic tissue rather than the F factor, which is an index to fibrosis, or the like.

### Citation List

### Patent Literature

Patent Literature 1: International Publication No. WO 2017-126514

### Non Patent Literature

Non Patent Literature 1: Suzuki A, Angulo P, Lymp J, Li D, Satomura S, Lindor K. Hyaluronic acid, an accurate serum marker for severe hepatic fibrosis in patients with non-alcoholic fatty liver disease. Liver Int 2005; 25:779-786.
Non Patent Literature 2: Tianhui L, Xiaoming W, Morten A, Diana J.L, and Federica G. Molecular Serum Markers of Liver Fibrosis. Biomark Insights. 2012; 7: 105-117.
Non Patent Literature 3: Kuno A, Ikehara Y, Tanaka Y, et al. A serum "sweet-doughnut" protein facilitates fibrosis evaluation and therapy assessment in patients with viral hepatitis. Sci Rep. 2013; 3:1065.

### Summary of Invention

### Technical Problem to be Solved by the Invention

A disease-specific noninvasive marker which enables evaluating the progress of hepatic fibrosis in NASH is needed to be found.

### Solution to the Problem

The present inventors have earnestly repeated investigation to achieve the above-mentioned object and found that the expression of a sugar chain (A2F bisect) and biosynthetic precursor sugar chains thereof in blood increase with the progress of hepatic fibrosis in NASH. The present inventors have also found that the A2F bisect sugar chain bound to IgA2 and the biosynthetic precursors of the A2F bisect sugar chain bound to IgA2 are preferable markers. The present inventors have completed the present invention based on this knowledge.

Accordingly, the present invention provides the following.
(1) A method for evaluating progress of hepatic fibrosis in NASH, comprising measuring an amount of a sugar chain having a structure represented by formula (I) : 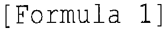 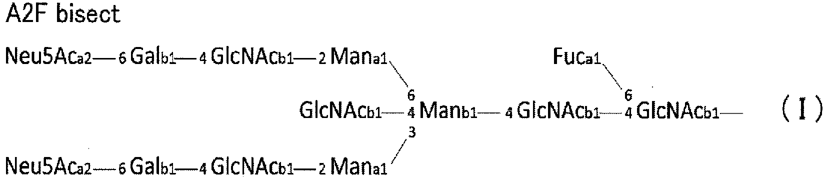 and/or a biosynthetic precursor sugar chain of the sugar chain having the structure represented by formula (I) in a sample.
(2) The method according to (1), comprising measuring a sum total of amounts of biosynthetic precursor sugar chains of the sugar chain having the structure represented by formula (I).
(3) The method according to (1) or (2), wherein the biosynthetic precursor sugar chain of the sugar chain having the structure represented by formula (I) has one or more structures selected from the group consisting of sugar chains 1, 2A, 2B, 3A, 3B, 4, 5A, and 5B: 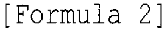 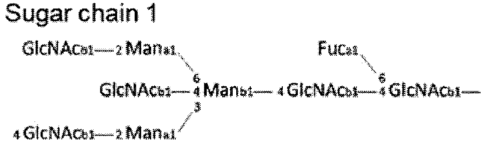 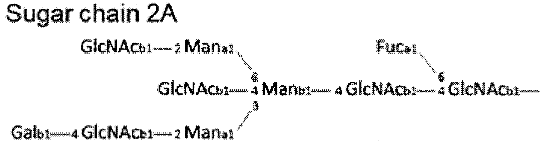 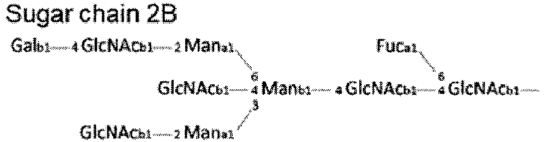 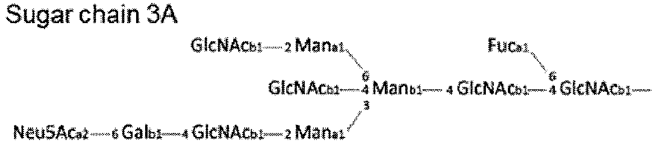 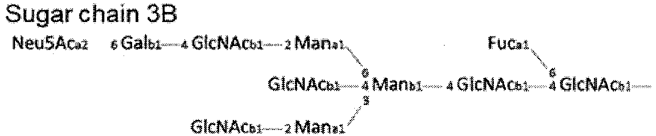 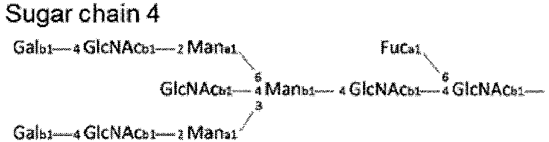 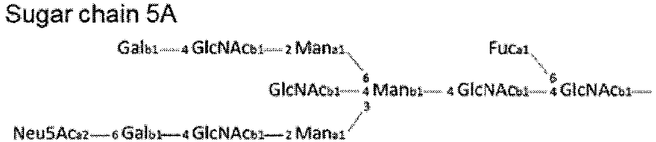 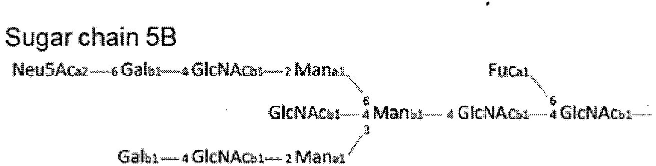
(4) The method according to any one of (1) to (3), wherein the sugar chain having the structure represented by formula (I) and/or the biosynthetic precursor sugar chain of the sugar chain having the structure represented by formula (I) is bound to IgA2.
(5) The method according to any one of (1) to (4), wherein the sample is a blood sample.
(6) A marker for evaluating progress of hepatic fibrosis in NASH, comprising a sugar chain having a structure represented by formula (I): 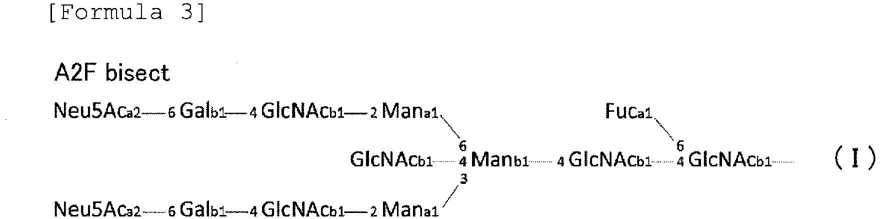 and/or a biosynthetic precursor sugar chain of the sugar chain having the structure represented by formula (I).
(7) The marker according to (6), wherein the biosynthetic precursor sugar chain of the sugar chain having the structure represented by formula (I) has one or more structures selected from the group consisting of sugar chains 1, 2A, 2B, 3A, 3B, 4, 5A, and 5B: 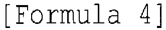 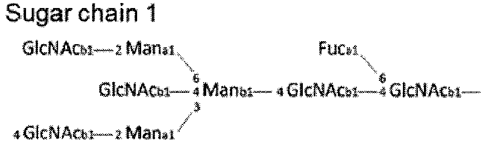 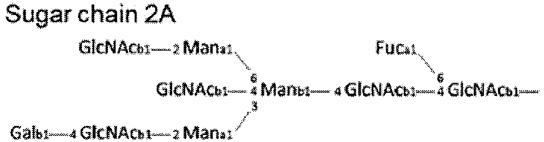 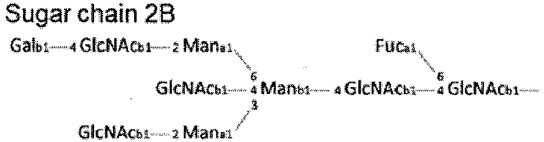 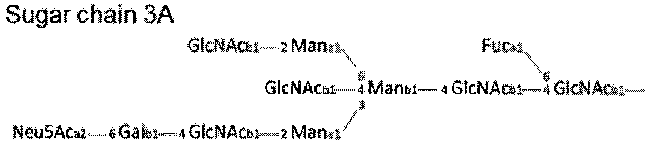 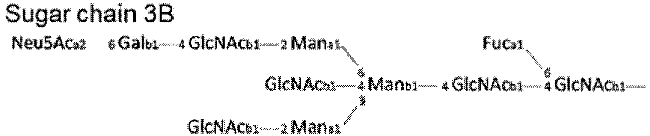 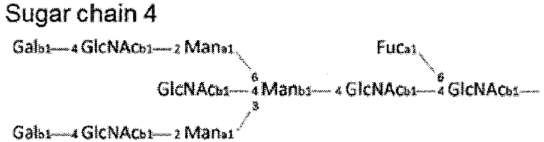 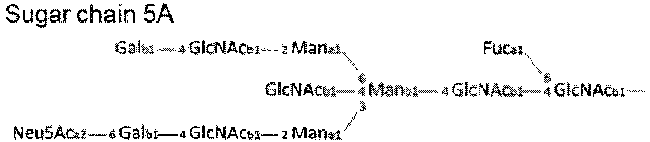 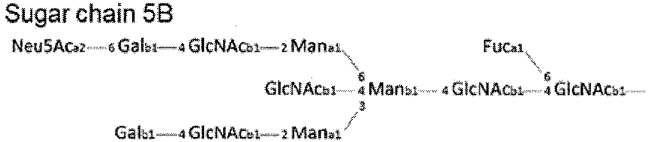
(8) The marker according to (6) or (7), wherein the sugar chain having the structure represented by formula (I) and/or the biosynthetic precursor sugar chain of the sugar chain having the structure represented by formula (I) is bound to IgA2.
(9) A kit for evaluating progress of hepatic fibrosis in NASH, comprising means for measuring an amount of a sugar chain having a structure represented by formula (I): 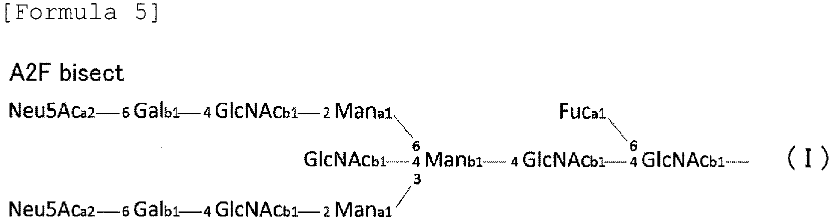 and/or a biosynthetic precursor sugar chain of the sugar chain having the structure represented by formula (I); and/or the sugar chain having the structure represented by formula (I) and/or the biosynthetic precursor sugar chain of the sugar chain having the structure represented by formula (I).
(10) The kit according to (9), wherein the biosynthetic precursor sugar chain of the sugar chain having the structure represented by formula (I) has one or more structures selected from the group consisting of sugar chains 1, 2A, 2B, 3A, 3B, 4, 5A, and 5B: 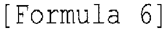 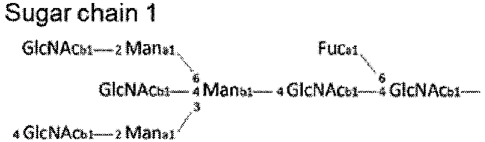 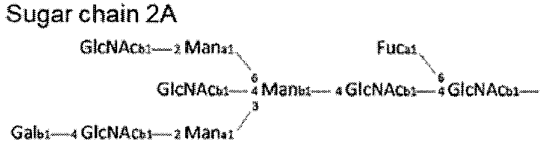 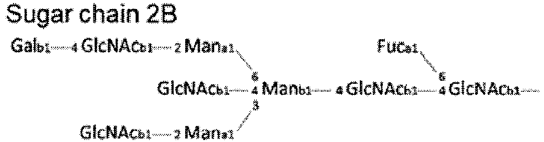 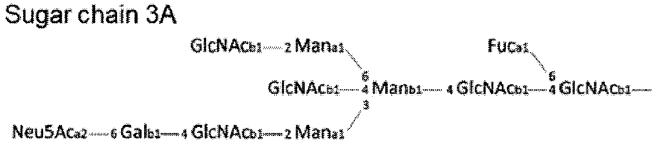 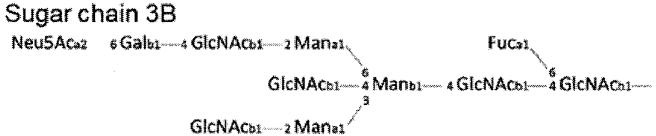 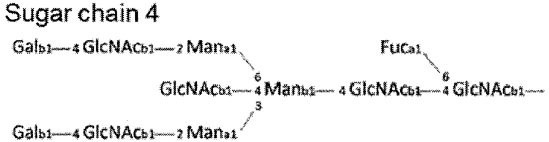 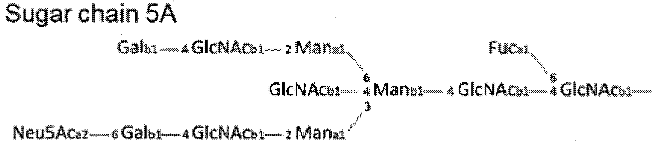 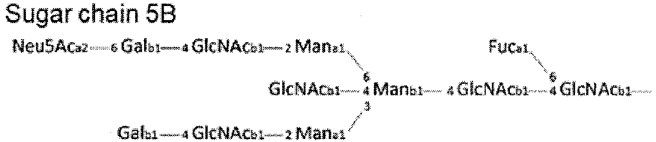
(11) The kit according to (9) or (10), wherein the means is an antibody or an antibody fragment specific to a protein to which the sugar chain having the structure represented by formula (I) and/or the biosynthetic precursor sugar chain of the sugar chain having the structure represented by formula (I) is bound; or a protein or a peptide which interacts with the protein.
(12) The kit according to (11), wherein the antibody or the antibody fragment, or the protein or the peptide which interacts is specific to IgA2.

### Effects of Invention

According to the present invention, a disease-specific noninvasive marker which enables evaluating the progress of hepatic fibrosis in NASH is provided. Accordingly, hepatic fibrosis in NASH can be determined correctly, and burdens on patients can also be reduced using the marker of the present invention. The progress degree of NASH and the therapeutic effect on NASH can be correctly investigated using the marker of the present invention.

### Brief Description of Drawings

[Figure 1] Figure 1 is a graph showing the expression amount of the A2F bisect accompanying fibrosis.
[Figure 2] Figure 2 is a graph showing changes in the expression of the biosynthetic precursor sugar chains of the A2F bisect accompanying fibrosis. The arrows in the figure show the biosynthetic pathway of A2F. The structures of a sugar chain 1, sugar chains 2 (2A, 2B), sugar chains 3 (3A, 3B), 4, and sugar chains 5 (5A, 5B) are shown herein.
[Figure 3] Figure 3 is a graph showing the results obtained by analyzing the correlation between pathological fibrosis evaluation in liver biopsies and the expressions of fibrosis evaluation factors and the A2F bisect in blood.
[Figure 4] Figure 4 is a graph comparing the expression amounts of the A2F bisect of NASH fiber groups with those of a hepatitis C (HCV) patient group.
[Figure 5] Figure 5 is a graph showing the result obtained by performing ROC analysis using samples the fibrosis stages of which are defined (F3 or more serious). The figure shows the ROC curves of IV type collagen 7s, Fib4, the A2F bisect, and the sum total of biosynthetic precursor sugar chains 1, 2A, 2B, 3A, 3B, 4, 5A, and 5B of the A2F bisect from left to right.
[Figure 6] Figure 6 shows the results obtained by analyzing N-linked sugar chains in the flow through and the protein G-bound fraction of NASH F3/4 pooled serum. The left panel is the results obtained by subjecting serum and the fractions to SDS-PAGE (CBB staining). The middle panel is analysis charts of the N-linked sugar chains by MALDI-TOF MS. The right panel is enlarged charts near an m/z of 3100 (highlighted areas).
[Figure 7] Figure 7 shows the results obtained by subjecting protein bands detected by CBB staining by separating protein G-bound fraction of NASH F3/4 pooled serum by SDS-PAGE to N-linked sugar chain analysis and carrier analysis.
[Figure 8] Figure 8 shows the results obtained by investigating the serum IgA2 concentrations accompanying fibrosis progress using an IgA2 ELISA kit.

### Description of embodiments

In a first aspect, the present invention provides a method for evaluating the progress of hepatic fibrosis in NASH, comprising measuring the amount of a sugar chain having a structure represented by formula (I): 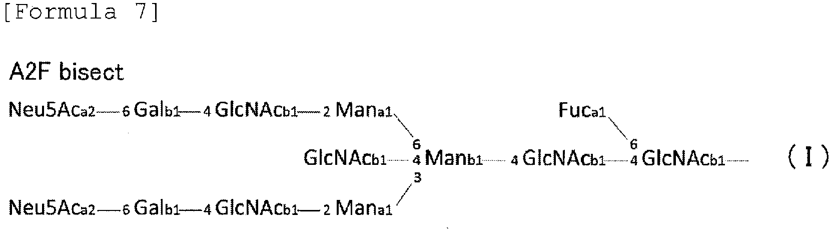 and/or a biosynthetic precursor sugar chain of the sugar chain having a structure represented by formula (I) in a sample.

The sugar chain having the structure represented by formula (I) is called the A2F bisect. The A2F bisect is an N-linked sugar chain, and is bound to proteins through asparagine residues. As long as the biosynthetic precursor sugar chain of the A2F bisect is a sugar chain located upstream of the biosynthetic pathway of the A2F bisect, the biosynthetic precursor sugar chain may be any sugar chain. Examples of the biosynthetic precursor sugar chain of the A2F bisect include sugar chains having structures represented by sugar chains 1, 2A, 2B, 3A, 3B, 4, 5A, and 5B: 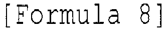 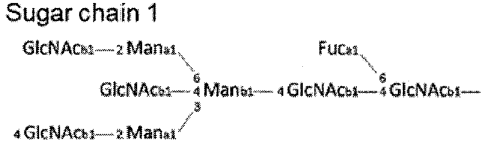 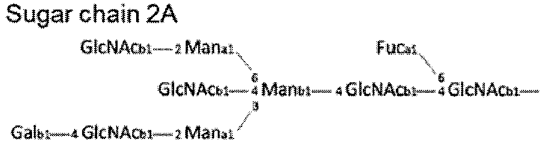 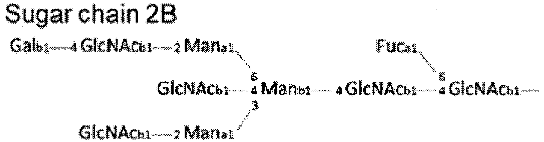 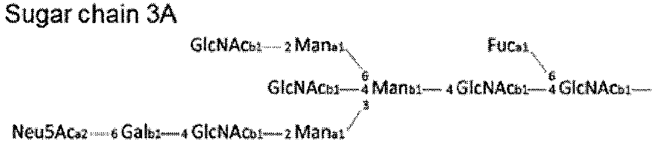 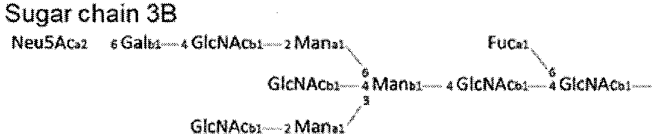 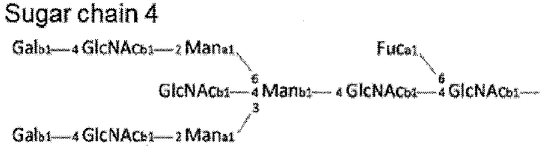 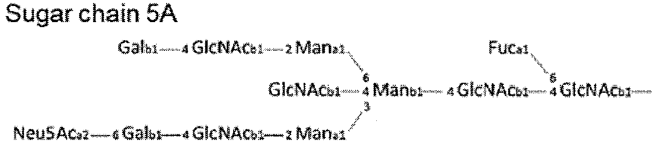 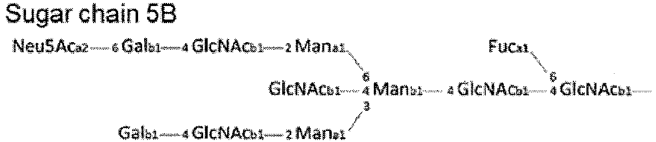 (these sugar chains may be called sugar chains 1, 2A, 2B, 3A, 3B, 4, 5A, and 5B for convenience herein). However, the biosynthetic precursor sugar chain is not limited to these.

In formula (I) and the sugar chains 1, 2A, 2B, 3A, 3B, 4, 5A, and 5B, GlcNAc represents N-acetylglucosamine, Man represents mannose, Fuc represents fucose, Gal represents galactose, and Neu5Ac represents N-acetylneuramic acid. In formula (I) and the sugar chains 1, 2A, 2B, 3A, 3B, 4, 5A, and 5B, a1-3, a1-6, and a2-6 represent an α1-3 glycoside bond, an α1-6 glycoside bond, and an α2-6 glycoside bond, respectively, and b1-2 and b1-4 represent a β1-2 glycoside bond and a β1-4 glycoside bond, respectively. In formula (I) and the sugar chains 1, 2A, 2B, 3A, 3B, 4, 5A, and 5B, GlcNAcb1-, which is a reducing terminal, represents a bond with asparagine in protein.

In the mentioned method of the present invention described above, the A2F bisect and/or the biosynthetic precursor sugar chain of the A2F bisect is used as a marker for hepatic fibrosis in NASH. Only the A2F bisect may be used as a marker, or one or more biosynthetic precursor sugar chains of the A2F bisect may be used as markers. Alternatively, the A2F bisect and one or more biosynthetic precursor sugar chains of the A2F bisect may be used as markers. The accuracy with which the progress of hepatic fibrosis is evaluated can be increased by determining the sum total of the amounts of two or more sugar chains used as markers. For example, the progress of hepatic fibrosis may be evaluated by determining the sum total of two or more biosynthetic precursor sugar chains of A2F bisect used as markers. In a first embodiment of the method of the present invention, the progress of hepatic fibrosis is evaluated by determining the amount of only the A2F bisect. In a further embodiment of the method of the present invention, the progress of hepatic fibrosis is evaluated by determining the sum total of the amounts of eight sugar chains which are the sugar chains 1, 2A, 2B, 3A, 3B, 4, 5A, and 5B.

The present inventors have found that the A2F bisect sugar chain modification in IgA2 is promoted accompanying the progress of hepatic fibrosis, while IgA2 in serum does not meanwhile increase significantly. Accordingly, in the present invention, preferable markers for hepatic fibrosis in NASH are the A2F bisect bound to IgA2 (immunoglobulin IgA2 protein) and biosynthetic precursor sugar chains of the A2F bisect bound to IgA2. It was not known in the past that the A2F bisect and the biosynthetic precursor sugar chains thereof can be used as markers for the progress of hepatic fibrosis in NASH, and that the A2F bisect bound to IgA2 and the biosynthetic precursor sugar chains of the A2F bisect bound to IgA2 can be used as preferable markers for the progress of hepatic fibrosis in NASH.

The IgA2 protein may be a wild type or a mutant. The IgA2 mutant may be, for example, a mutant produced by mutation and polymorphism in the body. The IgA2 mutant may have an amino acid sequence obtained by subjecting one to several amino acids in the amino acid sequence of wild type IgA2 to deletion, substitution, addition, or insertion. Here, the "several" means two, three, four, five, six, seven, eight, or nine. In the mutant IgA2 protein, preferably asparagine residues to which a sugar chain can be bound are conserved.

A sample used for the method of the present invention may be that obtained from any subject. The sample is preferably a sample obtained from a subject contracting NASH or a subject suspected of contracting NASH is preferable. A sample obtained by an invasive method such as liver biopsy is not needed. Examples of the sample include blood, urine, cerebrospinal fluid, lymph fluid, saliva, and sweat. A blood sample is preferable, and serum is more preferable.

The means and method for measuring the expression amounts (occasionally referred to merely as the amounts) of the A2F bisect and/or the biosynthetic precursor sugar chains thereof in a sample are well-known to those skilled in the art. For example, as described in Example 1, a protein fraction in serum is precipitated by a technique such as ethanol precipitation and purified to prepare a labeled sugar chain by glycoblotting and sialic acid linkage-specific amide labeling (SALSA). The amount of the sugar chain may be measured by analyzing the obtained labeled sugar chain with a MALDI-TOF mass spectrum. Analysis is performed using an absolute quantified value of each sugar chain detected by adding an internal standard sugar chain at a known concentration at the time of measurement, or a relative quantified value calculated from a total amount of the N-linked sugar chains in serum. For example, a protein to which the A2F bisect and/or a biosynthetic precursor sugar chain thereof are bound is separated using an antibody or an antibody fragment specific to the protein bound to the A2F bisect and/or the biosynthetic precursor sugar chain thereof or a protein or a peptide such as a peptide M which interacts with the protein bound to the A2F bisect and/or the biosynthetic precursor sugar chain thereof. The amount of the A2F bisect and/or the biosynthetic precursor sugar chain thereof bound to the protein may be measured, for example, using well-known chromatography, mass spectroscopy, or the like. The antibody may be either of a monoclonal antibody and a polyclonal antibody. A monoclonal antibody is preferable. In chromatography, a lectin to be bound to the A2F bisect and/or the biosynthetic precursor sugar chain thereof may be used.

The means and the method for measuring the amounts of the A2F bisect bound to IgA2 and the biosynthetic precursor sugar chains of the A2F bisect bound to IgA2 in the sample are also well-known. First, IgA2 in the sample is separated. For example, IgA2 may be separated using an anti-IgA2 antibody, a fragment of the antibody, or another protein or a peptide which interact with IgA2. IgA2 may be separated using immunoprecipitation or affinity chromatography. Next, the amounts of A2F bisect and/or the biosynthetic precursors thereof bound to the separated IgA2 are measured. The method for measuring the amounts of these sugar chains is as explained above.

In the method of the present invention, it can be estimated that as the amounts of the A2F bisect and/or the biosynthetic precursor sugar chains thereof in the sample is larger, the hepatic fibrosis of the patient has more progressed. On the contrary, it can be determined that as the amounts of the A2F bisect and/or the biosynthetic precursor sugar chains thereof in the sample is smaller, the hepatic fibrosis of the patient has less progressed. For example, the progress of hepatic fibrosis may be evaluated by comparing the amounts of the A2F bisect and/or the biosynthetic precursor sugar chains thereof in the sample derived from a subject not having hepatic fibrosis with the amounts of the above-mentioned sugar chains in a NASH patient sample. For example, as described in Example 4, the progress of hepatic fibrosis may be evaluated by determining a cutoff value using a ROC curve.

The evaluation of the progress of hepatic fibrosis includes determining at which stage the hepatic fibrosis is. Examples of the classification of hepatic fibrosis stages include the following new Inuyama classification: F0: No fibrosis, F1: Fibrous expansion in a portal vein region, F2: The formation of fibrous crosslink, F3: The formation of fibrous crosslink accompanied with lobe structure distortion, and F4: Hepatic cirrhosis.

In another aspect, the present invention provides a marker for evaluating the progress of hepatic fibrosis in NASH, comprising the A2F bisect and/or the biosynthetic precursor sugar chains of the A2F bisect. The A2F bisect and the biosynthetic precursor sugar chains thereof are as explained above.

The marker of the present invention may contain only the A2F bisect, may contain one or more biosynthetic precursor sugar chains of the A2F bisect, or may contain the A2F bisect and one or more biosynthetic precursor sugar chains of the A2F bisect. The first example of the marker of the present invention is a marker containing only the A2F bisect. The further specific example of the marker of the present invention is a marker containing seven sugar chains which are sugar chains 1, 2A, 2B, 3A, 3B, 4, 5A, and 5B.

Preferable markers of the present invention is the A2F bisect bound to IgA2 and the biosynthetic precursor sugar chains of the A2F bisect bound to IgA2.

The progress of hepatic fibrosis in the patient can be evaluated by measuring the amount of the marker of the present invention in the sample. Although the evaluation of hepatic fibrosis is as explained above, the evaluation will be described more specifically hereinafter. The method for evaluating the progress of hepatic fibrosis in NASH of the present invention includes comparing the amounts of the A2F bisect and the biosynthetic precursor sugar chains thereof on glycoprotein, the A2F bisect bound to IgA2, and the biosynthetic precursor sugar chains of the A2F bisect bound to IgA2 contained in blood with standard values. For example, when the amount of any of the A2F bisect and the biosynthetic precursor sugar chains thereof, and the A2F bisect bound to IgA2 and the biosynthetic precursor sugar chains of the A2F bisect bound to IgA2 in a subject is a threshold for determination with a healthy person group (standard value) or larger, hepatic fibrosis in NASH can be determined.

As mentioned above, the progress of hepatic fibrosis can be classified according to the classification: F0: No fibrosis, F1: Fibrous expansion in a portal vein region, F2: The formation of fibrous crosslink, F3: The formation of fibrous crosslink accompanied with lobe structure distortion, and F4: Hepatic cirrhosis. The method for evaluating the progress of hepatic fibrosis in NASH in the subject of the present invention enables determining at which stage among the above-mentioned F0 to F4 the liver of the subject is. In this case, the ranges of the standard values of subjects at various stages are measured beforehand. When the amount of any of the A2F bisect and the biosynthetic precursor sugar chains thereof, and the A2F bisect bound to IgA2 and the biosynthetic precursor sugar chains of the A2F bisect bound to IgA2 in a subject is in a specific range, the subject is likely at the corresponding stage. The classification is not limited to the above-mentioned classification of F0 to F4, and different classification may be used.

The accuracy with which the progress of hepatic fibrosis in NASH is evaluated can be increased using the marker of the present invention in combination with other hepatic fibrosis markers. Examples of the other hepatic fibrosis markers include, but not limited to, the IV type collagen 7S, hyaluronic acid, and M2BPGi.

In yet another aspect, the present invention provides a kit for evaluating the progress of hepatic fibrosis in NASH. The kit of the present invention is used for implementing the above-described method for evaluating progress of hepatic fibrosis in NASH. The kit of the present invention includes means for measuring the amounts of the A2F bisect and/or the biosynthetic precursor sugar chains of the A2F bisect; and/or the A2F bisect and/or the biosynthetic precursor sugar chains of the A2F bisect. The A2F bisect and the biosynthetic precursor sugar chains thereof are as explained above.

The means for measuring the amounts of the A2F bisect and/or the biosynthetic precursor sugar chains of the A2F bisect in the kit of the present invention is not particularly limited. The means may be means for separating the protein to which the A2F bisect and/or the biosynthetic precursor sugar chains of the A2F bisect are bound in the sample. Examples of such means include an antibody or an antibody fragment specific to the blood secreted protein to which the A2F bisect and/or the biosynthetic precursor sugar chains of the A2F bisect are bound, and a protein or a peptide such as a peptide M which interacts with the blood secreted protein. Special examples of the blood secreted protein to which the A2F bisect and/or the biosynthetic precursor sugar chains of the A2F bisect are bound include IgA2. The antibody may be either of a monoclonal antibody and a polyclonal antibody. A monoclonal antibody is preferable. Further examples of the means for measuring the amounts of the A2F bisect and/or the biosynthetic precursor sugar chains of the A2F bisect include a lectin which binds to the A2F bisect and/or the biosynthetic precursor sugar chains of the A2F bisect, and a carrier for chromatography containing such a lectin. Further examples of the above-mentioned means include reagents used for the glycoblotting method and sialic acid linkage-specific amide labeling (SALSA method).

Next, a method for measuring a marker will be described. The amounts of markers in blood can be measured by MS. For example, N-linked sugar chains are separated from serum by PNGase F digestion, and labeled sugar chains is prepared by glycoblotting and sialic acid linkage-specific amide labeling (SALSA). The labeled sugar chains can be analyzed by MALDI-TOF MS thereby. The labeled sugar chains can be quantified even without MS, for example, by cutting out a sugar chain component in blood plasma and making a lectin which specifically recognizes the A2F bisect and the biosynthetic precursor sugar chains thereof (E4-PHA) act on this. It is preferable to use a lectin labeled with an isotope element, a fluorescence reagent, or the like.

As the markers of the invention, the A2F bisect and the biosynthetic precursor sugar chains thereof bound to a specific protein or a specific peptide can be used. In this case, quantification can be performed by separating this fraction using an antibody selectively bound to the protein or the peptide to which the sugar chains are bound, and making this fraction act a lectin which specifically recognizes the A2F bisect and the biosynthetic precursor sugar chains thereof which exists on the protein. It is preferable to use a lectin labeled with an isotope element, a fluorescence reagent, or the like as the lectin. It is preferable to use IgA2 or IgM as the protein to which the sugar chains are bound.

Even though the above described fraction is not separated, a lectin or an antibody which recognizes the A2F bisect and the biosynthetic precursor sugar chains thereof bound to the specific protein or the specific peptide can also be used.

In yet another aspect, the present invention provides a method for evaluating the progress of hepatic fibrosis in NASH, comprising the following steps of:
(a) separating IgA2 or IgM in a sample and subsequently
(b) measuring the amounts of the A2F bisect and/or biosynthetic precursor sugar chains of the A2F bisect bound to IgA2 or IgM.

Example of the means for separating IgA2 or IgM contained in a sample include, but are not particularly limited to, an anti-IgA2 antibody or an anti-IgM antibody, a fragment of the antibody, and a protein or a peptide which interacts with IgA2 or IgM. These means can be suitably selected and used. The present inventors have found that protein G, which is originally used for the separation of IgG, can be suitably used for the separation of this purpose.

The method, the marker, and the kit of the present invention are specific to NASH, and enable evaluating the progress of hepatic fibrosis in NASH. The evaluation can also be noninvasively performed using the method, the marker, and the kit of the present invention.

The therapeutic effect on NASH may be evaluated using the method, the marker, and the kit of the present invention. Prognosis of NASH may be evaluated using the method, the marker, and the kit of the present invention. The efficacy of a therapeutic drug for NASH may be evaluated using the method, the marker, and the kit of the present invention. These evaluations can also be performed noninvasively.

Although the present invention will be described in more detail and specifically by showing the Examples hereinafter, the Examples do not limit the scope of the present invention.

The present application claims the priority to Japanese Patent Application 2019-129798, filed on July 12, 2019, and the entire content of Japanese Patent Application 2019-129798 is incorporated into the present application by reference.

### Example 1

To verify the usefulness of the A2F bisect and/or biosynthetic precursor sugar chains of the A2F bisect, N-linked sugar chains were comprehensively analyzed using serums at NASH F0, F1 or F2 (F1/F2), and F3 or F4 (F3/F4) (20 specimens, 22 specimens, and 28 specimens, respectively). Serum protein fractions were prepared by precipitating the serums with ethanol, and labeled sugar chains were prepared by glycoblotting and sialic acid linkage-specific amide labeling (SALSA). The labeled sugar chains were analyzed by MALDI-TOF MS.

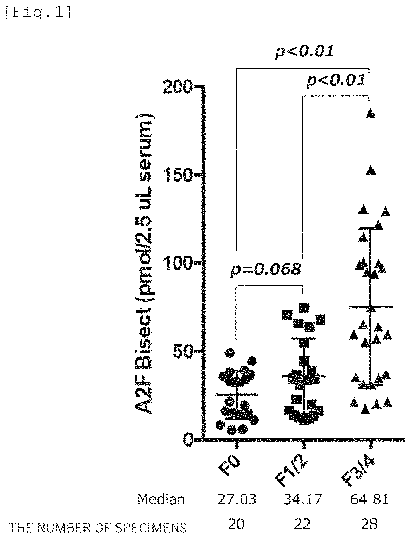

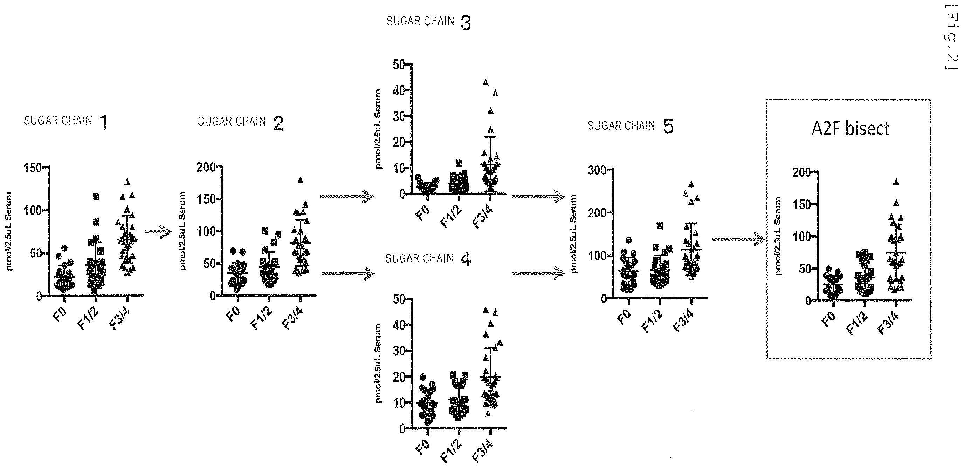

Figure 1 shows the results obtained by analyzing the A2F bisect by MALDI-TOF MS. It was confirmed that the expression amount of the A2F bisect increases accompanying the progress of fibrosis. In the sugar chains located upstream in the biosynthetic pathway of the A2F bisect, increase in the expression accompanying fibrosis was also confirmed in the similar way (Figure 2). In Figure 2, the sugar chain 1 to the sugar chain 5 are the sugar chain 1, the sugar chain 2 (2A and 2B), the sugar chains 3 (3A and 3B), 4, and the sugar chains 5 (5A and 5B), which were described above. Increase in the expression accompanying hepatic fibrosis was confirmed not only in the A2F bisect but also in the biosynthetic precursors thereof from these results.

### Example 2

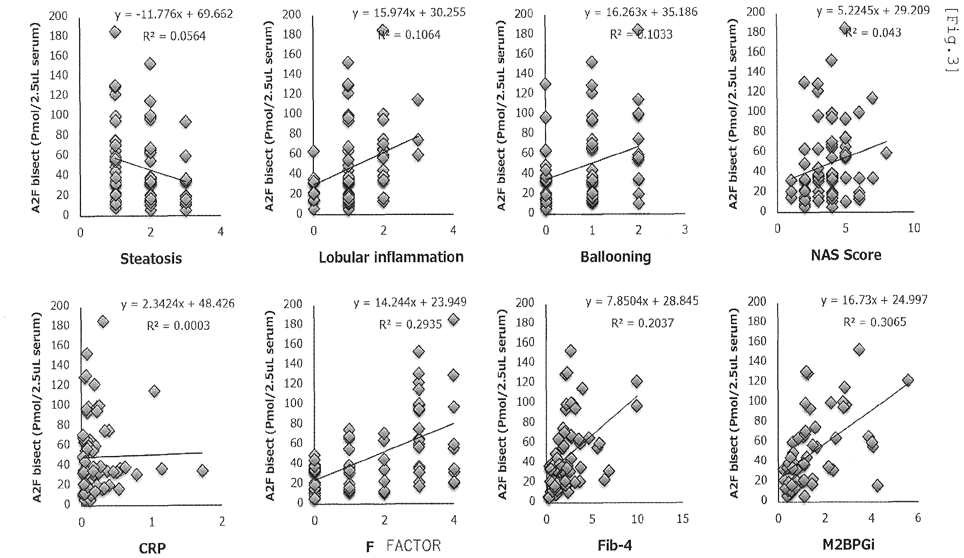

Next, the correlation between the expression amount of the A2F bisect and pathological fibrosis evaluation in liver biopsy and fibrosis evaluation factors in blood was analyzed (Figure 3). Consequently, while the expression amount of the A2F bisect exhibited high correlation with the F factor and the fib4 index, which are the indices to fibrosis, the expression amount of the A2F bisect did not exhibit correlation with CRP, which is used for evaluating inflammation, and exhibited negative correlation with the fatty liver factor (steatosis). It was confirmed from the above result that a change in the expression of the A2F bisect exhibited high correlation with fibrosis factors in NASH.

### Example 3

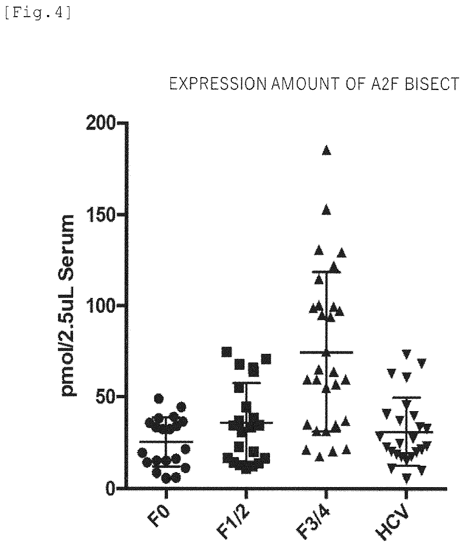

To examine the disease specificity of the A2F bisect sugar chain, the A2F bisect sugar chain in hepatitis C patient serums (HCV: 25 specimens) was measured by the similar technique, and the expression amount in the hepatitis C group was compared with the expression amounts in the NASH fiber groups (F0, F1/F2, and F3/F4) (Figure 4). Although the hepatitis C patients included cases in which hepatic fiber had progressed, in the hepatitis C patients, the expression amounts of the A2F bisect sugar chain did not exhibit high values unlike the NASH F3/4 group. In NASH, as fibrosis progressed, the expression amounts increased, and the expression of the A2F bisect sugar chain in the F3/4 group was twice or more the expression amount in the hepatitis C patients. Accordingly, it was suggested that the A2F bisect sugar chain was a disease specific marker which enables evaluating hepatic fibrosis progress in NASH disease.

### Example 4

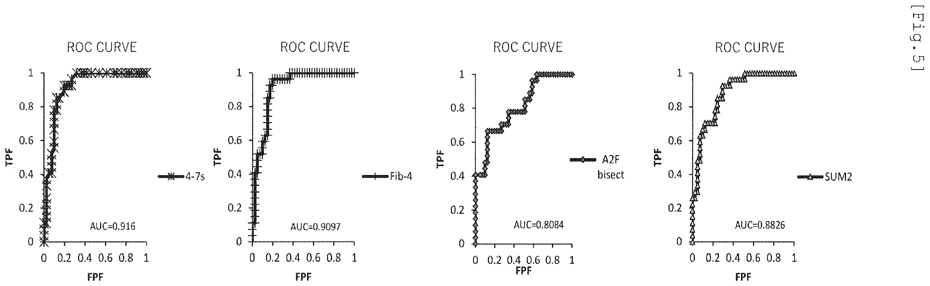

To evaluate the fibrosis identification sensitivity of the A2F bisect sugar chain, ROC analysis was performed using specimens the fibrosis stages of which were defined by liver biopsy and pathology diagnosis (F0 (20 specimens), F1/2 (22 specimens), and F3/4 (28 specimens)). Figure 5 shows the results. The AUC score increased using the sum total of the expression amounts of the precursor sugar chains shown in Figure 2 (the sugar chain 1, the sugar chains 2 (2A and 2B), the sugar chains 3 (3A and 3B), 4, and the sugar chains 5 (5A and 5B)) (SUM2) as compared with the expression amount of the A2F bisect sugar chain alone. It was revealed that the AUC score of this sum total was similar to the AUC score of IV type collagen 7s (described as 4-7s in Figure 5) or Fib-4. The difference in the shape of the curve between the sum total of the precursor sugar chains and the IV type collagen 7s was observed. It was shown that the analysis result (AUC score) by combining the sum total of the precursor sugar chains with IV type collagen 7s was significantly high as compared with the sum total of the precursor sugar chains alone and with IV type collagen 7s alone. The cutoff value of the diagnosis of serious fibrosis in the ROC curve of IV type collagen 7s was 6 ng/mL, and the false positive specimens exceeding this value were 5 specimens of 42 specimens (F1/2: 5 specimens). However, the false positive specimens by IV type collagen 7s could be reduced to 40% (3 specimens of 42 specimens) using the cutoff value of the total value of the A2F bisect and the biosynthetic precursor sugar chains 1, 2A, 2B, 3A, 3B, and 4 (139.7 pmol/2.5 µL serum).

### Example 5

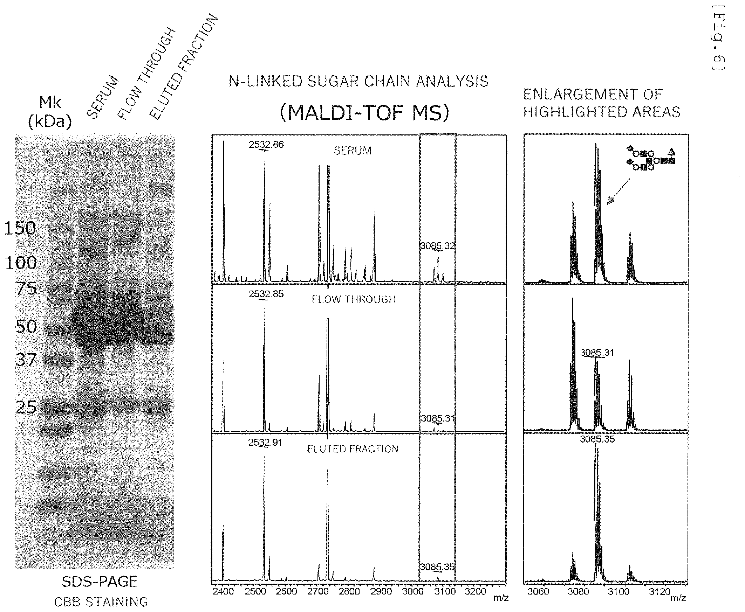

The following experiment was performed to identify a protein to which the A2F bisect sugar chain was bound. NASH F3/4 pooled serum was fractionated into flow through and a protein G-bound fraction (eluted fraction) with protein G. The fractions were confirmed by SDS-PAGE. The N-linked sugar chains of the flow through and the eluted fraction were analyzed by glycoblotting. Figure 6 shows the results. It was revealed that the A2F bisect sugar chain, the expression of which was observed to increase due to fibrosis of NASH, was contained in the eluted fraction in a large amount.

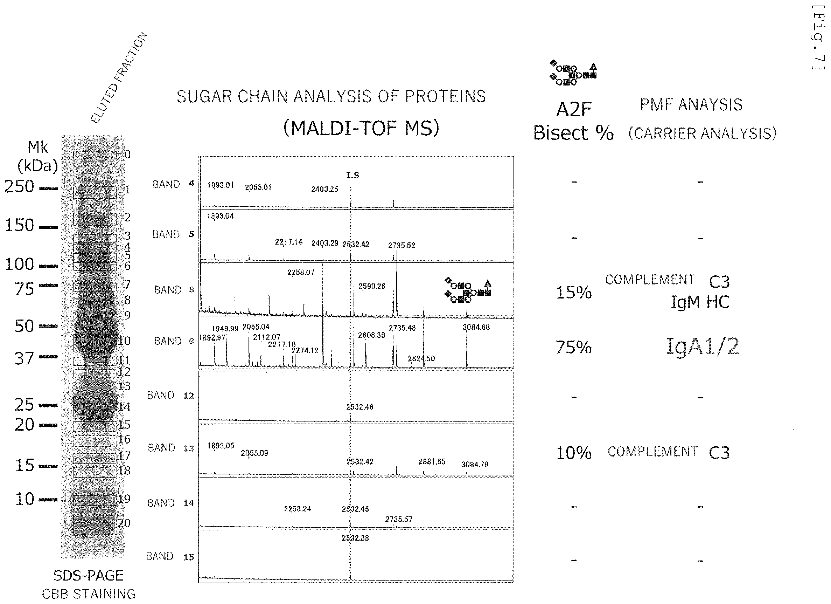

The eluted fraction by protein G in NASH F3/4 pooled serum was separated by SDS-PAGE, and protein bands detected by Coomassie brilliant blue (CBB) staining were subjected to N-linked sugar chain analysis. Figure 7 shows the results. The A2F bisect sugar chain was detected in the bands 8, 9, and 13. When the A2F bisect sugar chain was quantified with an internal standard sugar chain, the A2F bisect sugar chain was contained in the band 9 in the largest amount of 75%. The proteins contained in the bands were identified by peptide mass fingerprinting method (PMF), and it was revealed that complement C3 and IgM were contained in the band 8, and IgA was contained in the band 9.

The eluted fraction prepared from standard serum using protein G was digested with trypsin. A peptide to which the A2F bisect sugar chain was bound was comprehensively analyzed by LC-MS, and IgM and IgA2 in the IgA subclass were identified.

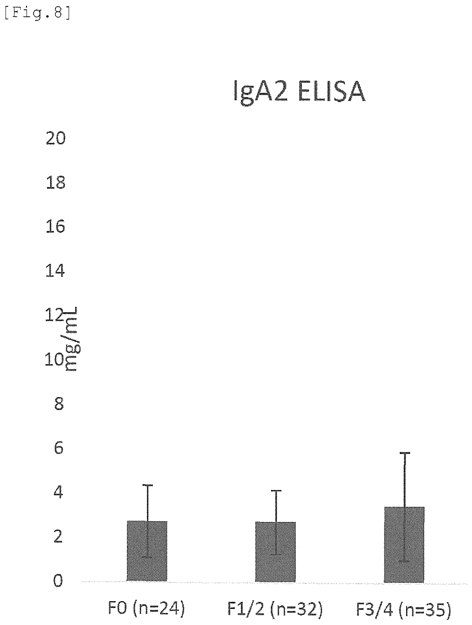

To evaluate the concentration of serum IgA2 in fibrosis progress, the concentrations of serum IgA2 in F0, F1/2, and F3/4 (20 specimens, 32 specimens, and 35 specimens, respectively) were measured using an IgA2 ELISA (enzyme-linked immunosorbent assay) kit. Figure 8 shows the results. While an increase in the concentration of serum IgA2 was seen accompanying the progress of fibrosis, a significant increase in the expression of IgA2 protein accompanying the progress of fibrosis was not seen. Accordingly, it was shown that the A2F bisect sugar chain modification in IgA2 was promoted with the progress of fibrosis.

The above results showed that the A2F bisect sugar chain bound to IgA2 was an effective biomarker for hepatic fibrosis in NASH.

### Industrial Applicability

The present invention is useful in the diagnosis of liver disease or the like. The present invention is useful especially in the evaluation of the progress of hepatic fibrosis in NASH. Accordingly, the present invention is useful in the field of diagnostic drugs for liver diseases, the field of research in liver diseases, and the like.

## Claims

1. A method for evaluating progress of hepatic fibrosis in NASH, comprising measuring an amount of a sugar chain having a structure represented by formula (I): 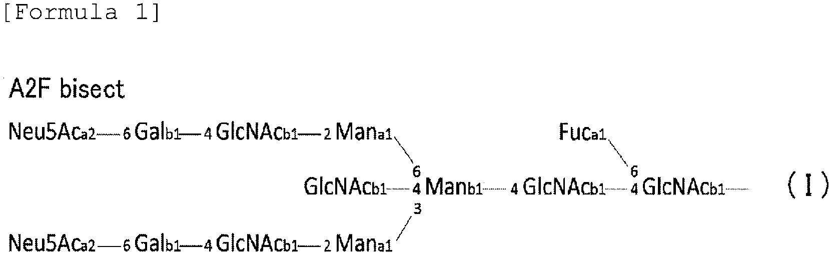 and/or a biosynthetic precursor sugar chain of the sugar chain having the structure represented by formula (I) in a sample.

2. The method according to claim 1, comprising measuring a sum total of amounts of biosynthetic precursor sugar chains of the sugar chain having the structure represented by formula (I).

3. The method according to claim 1 or 2, wherein the biosynthetic precursor sugar chain of the sugar chain having the structure represented by formula (I) has one or more structures selected from the group consisting of sugar chains 1, 2A, 2B, 3A, 3B, 4, 5A, and 5B:

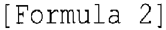

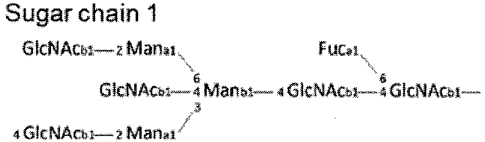

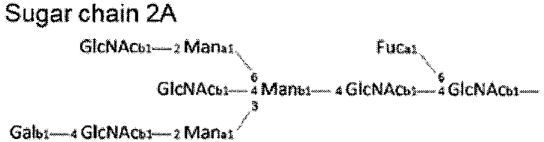

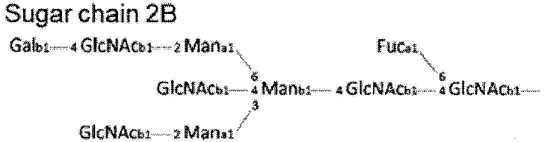

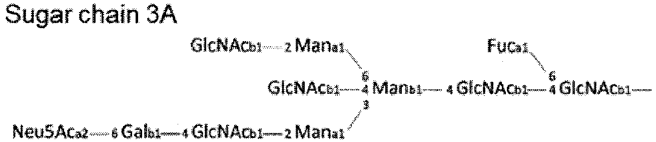

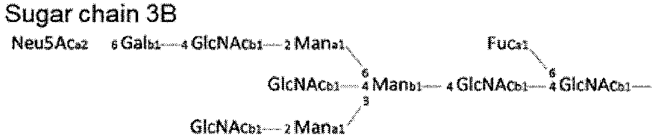

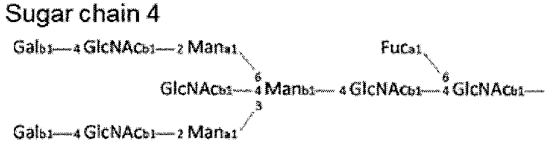

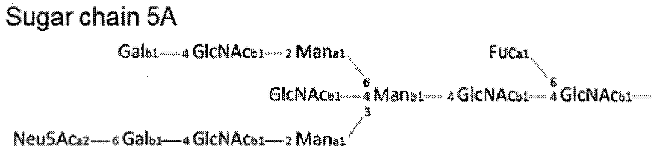

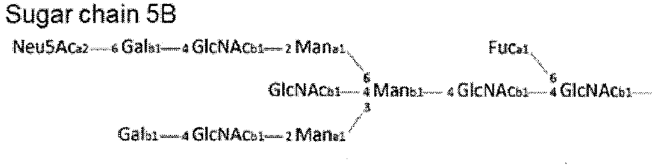

4. The method according to any one of claims 1 to 3, wherein the sugar chain having the structure represented by formula (I) and/or the biosynthetic precursor sugar chain of the sugar chain having the structure represented by formula (I) is bound to IgA2.

5. The method according to any one of claims 1 to 4, wherein the sample is a blood sample.

6. A marker for evaluating progress of hepatic fibrosis in NASH, comprising a sugar chain having a structure represented by formula (I): 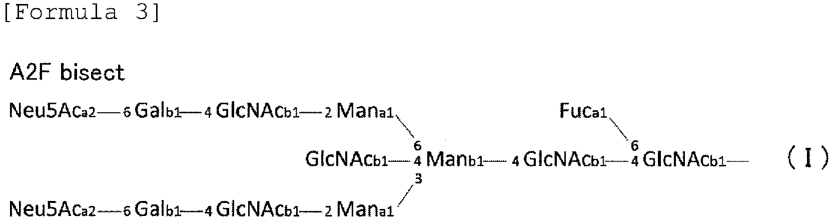 and/or a biosynthetic precursor sugar chain of the sugar chain having the structure represented by formula (I).

7. The marker according to claim 6, wherein the biosynthetic precursor sugar chain of the sugar chain having the structure represented by formula (I) has one or more structures selected from the group consisting of sugar chains 1, 2A, 2B, 3A, 3B, 4, 5A, and 5B:

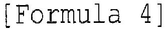

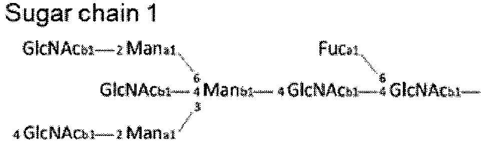

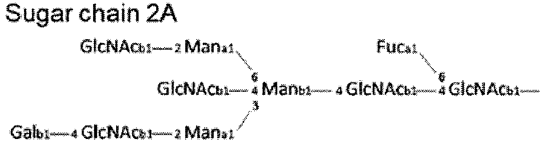

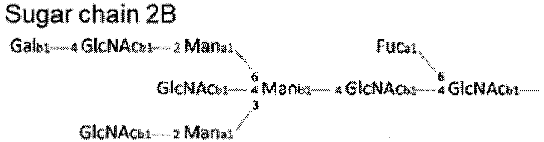

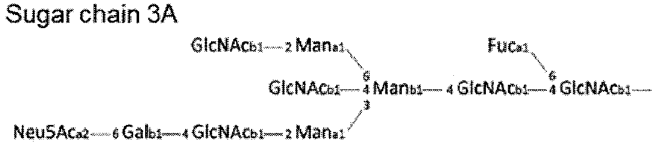

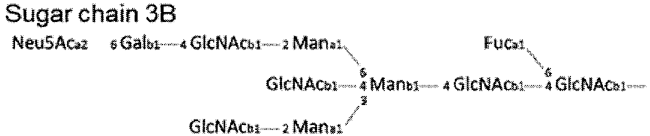

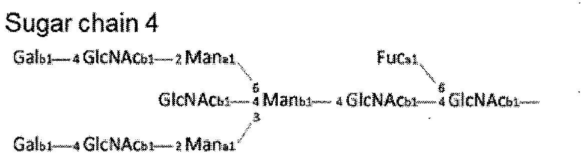

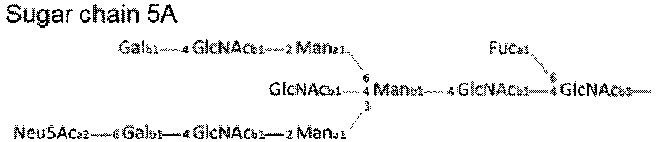

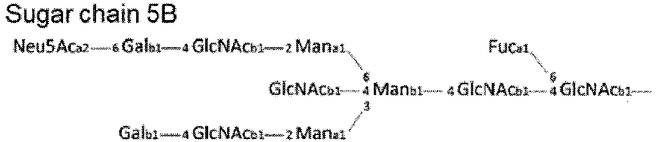

8. The marker according to claim 6 or 7, wherein the sugar chain having the structure represented by formula (I) and/or the biosynthetic precursor sugar chain of the sugar chain having the structure represented by formula (I) is bound to IgA2.

9. A kit for evaluating progress of hepatic fibrosis in NASH, comprising means for measuring an amount of a sugar chain having a structure represented by formula (I): 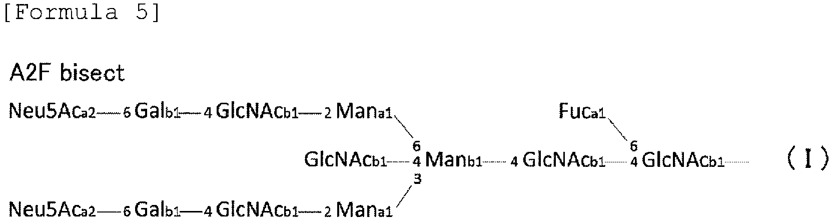 and/or a biosynthetic precursor sugar chain of the sugar chain having the structure represented by formula (I); and/or the sugar chain having the structure represented by formula (I) and/or the biosynthetic precursor sugar chain of the sugar chain having the structure represented by formula (I).

10. The kit according to claim 9, wherein the biosynthetic precursor sugar chain of the sugar chain having the structure represented by formula (I) has one or more structures selected from the group consisting of sugar chains 1, 2A, 2B, 3A, 3B, 4, 5A, and 5B:

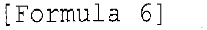

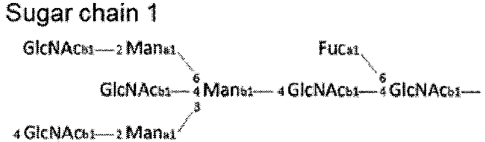

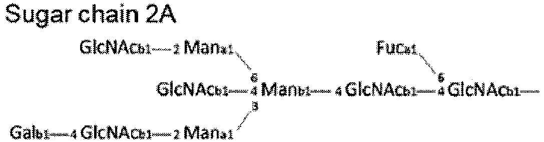

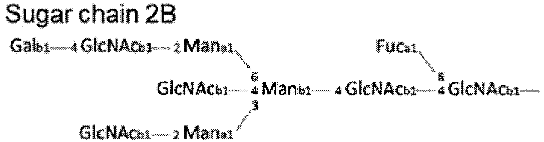

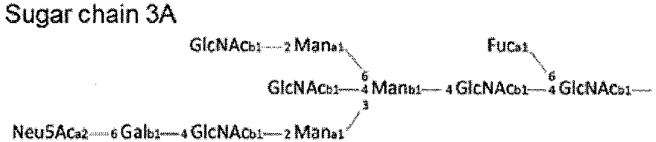

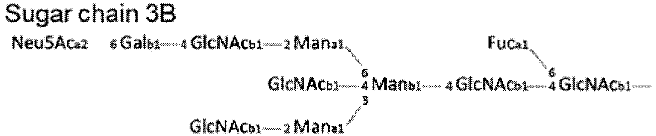

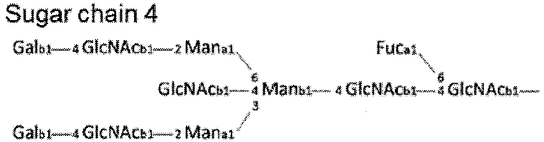

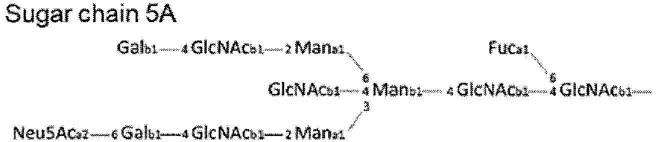

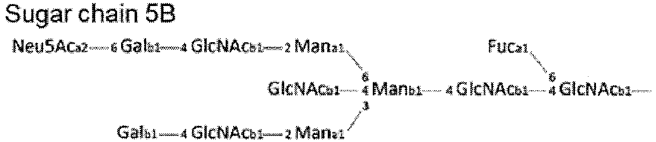

11. The kit according to claim 9 or 10, wherein the means is an antibody or an antibody fragment specific to a protein to which the sugar chain having the structure represented by formula (I) and/or the biosynthetic precursor sugar chain of the sugar chain having the structure represented by formula (I) is bound; or a protein or a peptide which interacts with the protein.

12. The kit according to claim 11, wherein the antibody or the antibody fragment, or the protein or the peptide which interacts is specific to IgA2.
